**Europäisches Patentamt**

**European Patent Office**

**Office européen des brevets**

(19)

(11) Veröffentlichungsnummer: **0 146 872**
**A2**

(12) # EUROPÄISCHE PATENTANMELDUNG

(21) Anmeldenummer: **84115223.4**

(22) Anmeldetag: **12.12.84**

(51) Int. Cl.⁴: **A 61 B 17/60**

(30) Priorität: 17.12.83 DE 3345726
28.06.84 DE 3423788
31.10.84 DE 3439795

(43) Veröffentlichungstag der Anmeldung:
03.07.85 Patentblatt 85/27

(84) Benannte Vertragsstaaten:
AT CH FR GB IT LI

(71) Anmelder: Schewior, Thomas, Dr.med.
Lessingstrasse 1
D-6903 Neckargemünd(DE)

(72) Erfinder: Schewior, Thomas, Dr.med.
Lessingstrasse 1
D-6903 Neckargemünd(DE)

(74) Vertreter: Geitz, Heinrich, Dr.-Ing.
Postfach 2708 Kaiserstrasse 156
D-7500 Karlsruhe 1(DE)

(54) **Vorrichtung zum Einrichten von Knochenabschnitten und/oder Knochenfragmenten.**

(57) Bei der Vorrichtung handelt es sich um einen sog. "Fixateur externe", der aus durch Stangen und Befestigungsmittel in ein- und verstellbaren Abständen miteinander verbindbaren Stellringen und aus Spanndrähten besteht, die sich in den von den Stellringen aufgespannten Ebenen erstrecken und mit ihren Enden mittels Halterungen an voneinander entfernten Teilen jeweils eines Stellrings unter Längsspannung befestigbar sind. Der Befestigung der Spanndrahtenden an den Stellringen dienen nachstellbare Spannelemente, die jederzeit während der Einsatzdauer eines derartigen Fixateurs ein Nachspannen der genannten Spanndrähte ermöglichen. In den Befestigungselementen sind die Stangen zum Verbinden benachbarter Stellringe stufenlos axialbeweglich und unabhängig davon innerhalb vorbestimmter Grenzen stufenlos winkelbeweglich gelagert, in allen Einstellagen jedoch arretiert.

EP 0 146 872 A2

./...

**Fig. 2**

842312

Anmelder: Schewior, Thomas, Dr. med.
Lessingstraße 1

D-6903 Neckargemünd

Vorrichtung zum Einrichten von Knochenabschnitten und/oder
Knochenfragmenten

========================================================

Die Erfindung bezieht sich auf eine Vorrichtung zum Einrichten von Knochenabschnitten und/oder Knochenfragmenten,
die aus durch Stangen und Befestigungselemente in
ein- und verstellbaren Abständen miteinander verbindbaren
Stellringen und aus mit letzteren unter Längsspannung
verbindbaren, sich in den von den Stellringen aufgespannten Ebenen erstreckenden und mit ihren Enden
mittels Halterungen an voneinander entfernten Teilen
jeweils eines Stellrings befestigbaren Drähten zur
Fixation der Knochenabschnitte oder -fragmente besteht.

Vorrichtungen dieser Art sind als sog. Fixateurs
externs (äußere Festhalter) bekannt und werden in
den medizinischen Disziplinen der operativen Orthopädie
der Gliedmaßen und der Unfallheilkunde zur Behandlung
von Gliedmaßenbrüchen verwendet. Eine mit Hilfe einer

-2-

derartigen Vorrichtung erzielte Fixation von Knochenteilen oder Knochenbruchstücken ist den verdeckten, an die zu verbindenden Knochenabschnitte direkt angepaßten und angeschraubten Platten insofern überlegen, als sie weichteilschonend ist und, fern von Knochenabschnitten oder Knochenbrüchen gelegen, angebracht wird sowie nachträglich ein- und nachstellbar ist.

Es sind einerseits uni-, bi- oder multilaterale Rahmenfixateure und andererseits zirkulär bzw. hemizirkulär angelegte Fixateure mit Ringen bzw. Ringabschnitten bekannt. Im Sinne der vorliegenden Erfindung werden nachstehend derartige Ringe oder Ringabschnitte ganz allgemein als "Stellringe" bezeichnet, auch wenn es sich dabei nicht um geschlossene Ringe, sondern um bügelartige Ringabschnitte handelt.

Fixateure aus Ringen bzw. Ringabschnitten oder entsprechenden Kombinationen aus Ringen und Ringabschnitten mit in jeder Ring- oder Ringabschnittsebene eingespannten paarigen gekreuzten, gelegentlich aber auch singulären, Drähten zur Knochenfragment-Fixation und aus diese Ebenen miteinander verbindenden Stangen sind den nichtringförmigen, etwa polygonalen Rahmenkonstruktionen insofern überlegen, als sie hohe Kräfte in den Ringebenen neutralisieren können und dank dieser Fähigkeit zur Kraftaufnahme die Montage der Knochen-Fixationsdrähte unter hoher Zugspannung ermöglichen, ferner aber auch dadurch, daß die die Knochenteile festhaltenden Drähte zum Zwecke der Spannbarkeit dünn und infolge der auf sie aufgebrachten Spannung stabil, gleichzeitig aber federnd sind, und daß die dünnen Drähte gewebeschonend und bei Bedarf auch sekundör austauschbar sind, ohne im Falle eines solchen Austauschs gegen neu zu bohrende Drähte die angebohrten Knochen bei wiederholter Drahtbohrung in ihrer Stabilität zu schwächen, und nicht nur zur Knochenfixation, sondern auch zu Manövern der Stellungskorrektur von Knochenbruchstücken und zur Erzeugung von Druck im Bruchspalt zwischen zwei einzurichtenden Knochen miteingesetzt werden können.

Unbefriedigend bei diesen Fixateuren ist allerdings, daß ihre dreidimensionale Anpassungsfähigkeit an eine vorgegebene anatomische Situation oder eine sich im Laufe der Behandlung verändernde Stellung der Fragmente beschränkt ist. Es ist der Grundkonstruktion nach nur das fluchtende Weiter- oder Engerstellen der Abstände der parallelen Ring- bzw. Ringabschnittsebenen möglich. Andere als planparallele Ringebenen sind nur durch zusätzlichen, umständlichen, in der stereometrischen Anordnung und Funktion nicht sicher bestimmbaren Einbau von kreuzgelenkartigen Ankopplungselementen möglich, wobei jedes dieser Elemente das Gewicht des äußeren Festhalters erhöht und seine Gesamtstabilität verringert.

Während der Behandlung ist meist ein sukzessives Verstellen der relativen Lage der Stellringe und damit auch der von ihnen gehaltenen und geführten Knochenabschnitte notwendig, sei es, daß dabei eine primär gegebene Distanz (Dislokation) von Knochenfragmenten zu beseitigen ist, sei es, daß senkundäre durch Gewebeanspannung unerwünschte Lageveränderungen von Knochenabschnitten zu korrigieren sind, oder sei es, daß therapeutisch beabsichtigte plastische Verformungen des gesamten Gliedmaßenknochens in Form von Verlängerungen, Verkürzungen oder Achsenkorrekturen vorgesehen sind.

Eben diese Repositionen oder plastischen Knochenverformungen (Richtarbeiten) sind aber mit herkömmlichen Festhaltern nur umständlich und unsicher, unvollständig und unter Stabilitätsverlust durchführbar. An sog. windschiefen räumlichen Knochenfragmentkonstellationen kann mit bekannten Ringfixateuren noch gar keine anatomisch befriedigende Positionsänderung erzielt werden.

Unbefriedigend bei diesen externen Fixateuren ist auch, daß die in den Ring- oder Ringabschnittsebenen gespannten, die auszurichtenden Knochenabschnitte in aller Regel entfernt von einer Fraktur durchdringenden Drähte nur mittels besonderer Spannmittel spannbar und dann mit ihren Enden an voneinander entfernten Abschnitten eines Stellrings festlegbar sind, etwa infolge Einklemmung zwischen Bereichen des Stellrings und einer an diesem angebrachten Klemmschelle. Als nachteilig hat sich ferner erwiesen, daß die jeweils im Abstand von einer Fraktur plazierten Drähte eine den Durchmesser der Stellringe überschreitende Länge aufweisen müssen, um mittels geeigneter Halterungen unter einer vorbestimmten Längsspannung an voneinander entfernten Abschnitten jeweils eines Stellrings befestigbar zu sein, wobei die radial über einen Stellring hinausragenden Drahtabschnitte zum Zwecke der Aufbringung einer Längsspannung beispielsweise
mittels einer Spannzange erfaßtbar sein müssen.

Demgegenüber besteht die Zielsetzung der Erfindung
in der Verbesserung der eingangs erläuterten Vorrichtung
zum Einrichten von Knochenabschnitten und/oder Knochenfragmenten dahingehend, daß dreidimensionale Formveränderungen des äußeren Festhalters und damit Positionsänderungen der in ihrer Lage zu korrigierenden Knochenfragmente stufenlos möglich werden und eine stufenlose
Arretierung in jeder Einstellage gelingt, wobei die
Gesamtstabilität auch während eines jeden Teilmanövers
einer Knochenfragment-Positionsänderung beibehalten
wird und einzelne oder bedarfsweise kombinierte Freiheitsgrade von Korrekturbewegungen der Knochenfragmente im Bedarfsfalle auch selektiv ermöglicht werden
sollen.

Ferner besteht die der Erfindung zugrundeliegende
Aufgabe in der Verbesserung der eingangs erläuterten
Vorrichtung dahingehend, daß die in einer Ring- oder
Ringabschnittsebene plazierten und einzurichtende
Knochenabschnitte durchdringenden Drähte in einfacher
Weise an den Stellringen befestigbar und spannbar
sind und daß während der gesamten Behandlungsdauer
die Drahtspannung leicht variierbar und dementsprechend
die Stabilität der Gesamtanordnung an den jeweiligen
Behandlungsfall anpaßbar ist.

Ausgehend von der Vorrichtung nach dem Oberbegriff
des Patentanspruchs 1 ist die erste Teilaufgabe der
Erfindung dadurch gelöst, daß die Stangen mittels
der Befestigungselemente innerhalb vorbestimmter
Grenzen allseitig schwenkbar an den Stellringen gelagert und in jeder Schwenkstellung arretierbar sind, und
daß die Abstände zwischen benachbarten Stellringen
unabhängig von den eingestellten Schwenklagen der
Stangen einstellbar sind.

Eine Ausgestaltung dieser Vorrichtung ist dadurch
gekennzeichnet, daß die Stellringe mit sich senkrecht
zu den von ihnen aufgespannten Ebenen erstreckenden
und über den Umfang verteilt angeordneten Ausnehmungen
für die austauschbare Aufnahme der Befestigungselemente
versehen sind, und daß die in diesen Ausnehmungen
fest, jedoch lösbar montierten Befestigungselemente
sphärisch konfigurierte, in jeder Einstellage stufenlos arretierbare Schwenklagerungen sowie stufenlos
verstellbare Längslagerungen für die Stangen aufweisen.

Angesichts der stufenlosen Verschwenkbarkeit der Stangen
in den sphärisch konfigurierten Schwenklagern und
der Stangenlängsbeweglichkeit gelingen allen Erfordernissen bei bestimmungsgemäßer Verwendung genügende
Korrekturmanöver und eine Fixation der Stangen in
den jeweils erzielten Einstellagen. Dadurch wird
eine allseitige Verformbarkeit eines erfindungsgemäß
aufgebauten Fixateurs erreicht, die den Vorteil hat,
daß der Operateur bei den je Stellringetage vorzunehmenden Drahtbohrungen sich nach den anatomischen
Gegebenheiten richten und dabei beispielsweise Gefäß-,
Nerven- und Sehnenverläufe schonen kann und bei der
Einstellung der Perforationsrichtung die Planparallität
zu benachbarten Stellringetagen nicht streng einzuhalten braucht. Wenn aus anatomischen Zwängen zueinander
geneigte oder parallel verschobene Stellringetagen resultieren, können je zwei benachbarte Ringebenen
in diesen sich zwangsläufig ergebenden Lagen mit
Hilfen der schwenkbaren und längeveränderlichen Stangenlagerungen spannungsfrei miteinander verbunden werden.

Zweckmäßigerweise besitzt bei der vorgenannten Ausgestaltung der Erfindung jede Schwenklagerung eine in
einer sphärische Lagerflächen aufweisenden Lagerschale aufgenommene Lagerkugel, durch die sich eine
Stange hindurcherstreckt, desgleichen eine mittels
Schraubgewinde mit der Lagerkugel verspannbare und
letztere in der Spannlage arretierende Klemmeinrichtung,
so daß in Abhängigkeit von dem Anspannen der Klemmeinrichtung infolge Betätigung des Schraubgewindes eine
jederzeit leicht lösbare, aber sicher wirkende Arretierung
in der jeweiligen Einstellage erreichbar ist. Als besonders vorteilhaft hat sich erwiesen, wenn die Lagerschale
mittels eines hülsenartigen Abschnittes, der in die
das Befestigungselement aufnehmende Ausnehmung im Stellring hineinragt, in letzterem fest, jedoch lösbar
verankert ist. Demgemäß kann im Bereich einer jeden
sich senkrecht zur Stellringebene durch den Stellring hindurcherstreckenden Ausnehmung ein erfindungsgemäßes Befestigungselement montiert werden. Der
hülsenartige Abschnitt kann ein Gewindeabschnitt
und in ein Innengewinde der Ausnehmung eingeschraubt
sein.

Eine andere Ausgestaltung sieht vor, daß die Klemmeinrichtung zum Arretieren der Lagerkugel einen
mittels eines Schraubgewindes relativ zu der die
Lagerkugel aufnehmenden Lagerschale einstellbaren
und in Abhängigkeit von seiner Einstellung an der Lagerkugel angreifenden Klemmring umfaßt. Dieser Klemmring kann mit seinem Schraubgewinde in ein Innengewinde der die Lagerschale aufnehmenden Ausnehmung im Stellring eingeschraubt sein.

Die Anordnung kann, gemäß einer anderen Ausgestaltung, aber auch so getroffen sein, daß der in die Ausnehmung im Stellring hineinragende Abschnitt der Lagerschale eine Länge größer als die Dicke des Stellringes aufweist und wenigstens auf dem im eingesetzten Zustand der Lagerschale auf der von letzterer entfernten Seite über die Stellringebene hinausragenden Teil mit Außengewinde versehen ist. Bei einer derartigen Ausbildung dieses Abschnittes kann der Klemmring auf den sich durch die Ausnehmung im Stellring hindurcherstreckenden Gewindeabschnitt der Lagerschale aufgeschraubt sein, desgleichen aber auch eine die eingeschraubte Lagerschale in ihrer Montageposition zusätzlich sichernde Kontermutter. Diese Kontermutter kann auch, nach einer gleichfalls weiteren Ausgestaltung, mit einem sich auf der von der Lagerschale entfernten Seite forterstreckenden Gewindeabschnitt versehen und auf letzteren der Klemmring aufgeschraubt sein.Die Lagerschale kann aber auch, nach einer anderen Ausgestaltung mit dem genannten Abschnitt in die Ausnehmung im Stellring einsteckbar und durch formschlüssig mit der Ausnehmung zusammenwirkende Mittel, wie Nut und Feder, gegen Drehung gesichert sein.

Gemäß einer anderen Ausgestaltung ist zwischen dem Klemmring und der Lagerkugel eine mittels des Klemmringes gegenüber der Lagerkugel verspannbare Klemmhülse mit einem sich von der Lagerkugel aus erweiternden und innerhalb eines vorbestimmten Winkelbereichs Schwenkbewegungen der Lagerkugel mit der sich durch diese hindurcherstreckenden Stange zulassenden Innenkonus angeordnet. Zweckmäßigerweise kann die Klemmhülse mit dem sich von der Lagerkugel aus öffnenden Innenkonus mit dem Klemmring drehbar, aber axialfest verbunden sein, wobei die Drehlagerung zwischen Klemmhülse und Klemmring reibungsarm gestaltet sein sollte, so daß beim Anziehen des Klemmrings die Klemmhülse infolge ihres reibungsschlüssigen Kontaktes mit der Lagerkugel bezüglich letzterer drehfest gehalten ist und die demgegenüber drehbare Klemmhülse im wesentlichen nur in bezug auf die in der Lagerkugel aufgenommene Stange axial gerichtete Spannkräfte auf die Lagerkugel aufbringt.

Diese Ausbildung der Klemmvorrichtung gewährleistet eine äußerst einfache Einstell- und Feststellbarkeit der Schwenklagerung. Analog dazu sieht eine andere Ausgestaltung der Erfindung vor, daß die sich durch die Lagerkugel hindurcherstreckende Stange als Gewindestange ausgebildet und in einer in der Lagerkugel drehbar, aber axialfest gelagerten Gewindehülse aufgenommen ist, so daß eine translatorische Stangeneinstellung in einfacher Weise durch Drehung der Gewindehülse gegenüber der Lagerkugel unabhängig von der jeweils eingestellten Schwenklage gelingt. Zweckmäßigerweise kann dabei die in der Lagerkugel drehbar, aber axialfest aufgenommene Gewindehülse im Bereich eines sich in Axialrichtung über die Klemmeinrichtung hinauserstreckenden Teils mit einem Rändelring bzw. einer Schlüsselansatzfläche zum Aufbringen einer Drehbewegung um die Gewindehülsenlängsachse versehen sein. In entsprechender Weise können die
sich zwischen benachbarten Stellringen erstreckenden
Gewindestangen mit Ansatzflächen zur Aufbringung
von Drehmomenten bzw. zum Festhalten gegen Drehung
versehen sein, so daß in einfacher Weise ein Mitdrehen
der Gewindestangen bei deren Längeneinstellung infolge
Drehung der in der Lagerkugel aufgenommenen Gewindehülse unterbunden werden kann.

Die zweite Teilaufgabe der Erfindung ist, ausgehend
vom Oberbegriff des Patentanspruchs 16, dadurch gelöst,
daß jeweils wenigstens eine der Halterungen zum Verbinden der Enden eines Spanndrahtes mit einem Stellring
als in Drahtlängsrichtung nachstellbares Spannelement
ausgebildet ist. Durch diese Ausbildung wenigstens
einer die Enden eines Spanndrahtes an einem Stellring
festlegenden Halterungen als nachstellbares Spannelement gelingt es, eine dem jeweiligen Anwendungsfall entsprechende Drahtspannung und demgemäß eine
dieser Drahtspannung entsprechende Stabilität in
jeder Ring- oder Ringteilebene zu erzeugen sowie
ggf. im Verlaufe einer Behandlung notwendig werdende
Korrekturen der Drahtspannung durchzuführen.

Eine zweckmäßige Weiterbildung des Spannelements
sieht vor, daß dieses in einer sich radial erstreckenden
Führung des Stellrings bewegbar aufgenommen und mittels
eines Einstellgewinde längs der Führung nachstellbar
ist, womit in Abhängigkeit von der Steigung des Einstellgewindes eine äußerst präzise Feineinstellung
der jeweils auf einen in einer Ring- oder Ringteilebene gespannten Draht aufbringbaren Drahtspannung
und damit auch eine Feindosierung etwaig notwendiger Korrekturen einer anfänglich erzielten Einstellung
von Knochteilen, beispielsweise im Bereich einer
Fraktur, ermöglicht ist.

Obgleich eine gewisse Verdrillung der Drähte sich
durchaus stabilitätsfördernd auswirken kann, ist
es im Interesse einer definierten Drahtspannung von
Vorteil, wenn nach einem anderen Ausgestaltungsmerkmal
der Erfindung das Spannelement verdrehfest in der
Führung des Stellrings aufgenommen ist, so daß Spannkräfte ausschließlich über das Einstellgewinde der
Spannvorrichtung auf einen in einer Ring- oder Ringteilebene gespannten Draht aufbringbar sind.

Eine besonders einfache Ausbildung des Spannelementes
ergibt sich, wenn dieses mit einer Gewindespindel
versehen und auf letzterer eine sich an einem Widerlager
des Stellrings abstützende Spannmutter aufgenommen
ist, so daß durch Drehung der Spannmutter nach der
einen oder anderen Richtung eine Erhöhung oder Reduzierung
der Drahtspannung gelingt.

Wenn nach einem anderen Ausgestaltungsmerkmal der
Erfindung die Spannelemente als Hülsen mit einer
sich radial zum Stellring erstreckenden Mittelbohrung
zum Aufnehmen eines Drahtendes ausgebildet und mit
Mitteln zum Festlegen der Drahtenden versehen sind,
gelingt eine besonders einfache und patientenfreundliche
Montage der Vorrichtung, indem bereits lagerichtig
für eine Ring- oder Ringteilebene , etwa beidseitig
einer Bruchstelle, gesetzte Drähte mit ihren Enden
in einander gegenüberliegend an einem Stellring aufgenommene Spannelementhülsen eingeführt und an diesen
so festgelegt werden, daß die Spannelementhülsen

in denaie aufnehmenden Führungen des Stellrings zwecks
Aufbringung der Drahtspannung radial nach außen bewegbar
geführt sind. Ein Vorteil dieser Ausbildung besteht
darin, daß die Länge der sich in einer Ring- oder
Ringteilebene erstreckenden Drähte kleiner als der
Ringdurchmesser sein kann, so daß keine Drahtenden
radial über die Stellringe hinausragen.

Als Mittel zum Festlegen der Drahtenden hat es sich
bei der Ausbildung der Spannelemente als Hülsen mit
sich radial erstreckenden Mittelbohrungen als zweckmäßig
erwiesen, wenigstens eine in einer rechtwinklig zur
Mittelbohrung der Spannelementhülse verlaufenden
Gewindebohrung aufgenommene Klemmschraube vorzusehen,
mittels der nach dem Einführen eines Drahtendes in
die genannte Mittelbohrung der Spannelementhülse ein
Abschnitt des Drahtendes innerhalb der Mittelbohrung
durch Klemmung gehalten wird. Anstelle einer Klemmschraube können selbstverständlich auch mehrere in
Richtung der Längsbohrung in der Spannelementhülse
im Abstand voneinander angeordnete Klemmschrauben
vorgesehen sein, so daß auch bei hoher Längsspannung
der Drähte deren zuverlässige Verbindung mit dem
Stellring gewährleistet ist.

Bei paariger und gekreuzter Anordnung der für eine Ring-
oder Ringteilebene vorgesehenen Drähte durchdringen
diese die einzurichtenden Knochenabschnitte in aller
Regel im Abstand voneinander. Angesichts dieser Anordnung der Drähte hat es sich als vorteilhaft erwiesen, wenn der Stellring in zwei beabstandeten
Parallelebenen mit in Umfangsrichtung in Abständen
voneinander angeordneten Aufnahmen für die Spannelemente zum Verbinden der Drahtenden mit dem Stellring versehen ist. Bei den Aufnahmen für die Spannelemente kann es sich um den Stellring radial durchbrechende Bohrungen handeln oder auch um jeweils zu einer Stellringseite offene Ausnehmungen, so daß nach der Befestigung der Drahtenden an den Spannelementen diese von den Stirnseiten des Stellringes aus in die Ausnehmungen des Stellringes einhängbar sind, was eine besonders einfache Montage ermöglicht.

Eine weitere wichtige Ausgestaltung sieht vor, daß der Stellring als, im Querschnitt gesehen, T-Profil ausgebildet ist, dessen Mittelsteg sich in einer Ebene radial nach außen erstreckt und bei dem die in beabstandeten Parallelebenen angeordneten Aufnahmen für die Halterungen sich in den Flanschteilen beidseitig des Mittelstegs erstrecken, was einerseits dem Stellring außerordentlich große Steifigkeit verleiht und andererseits ermöglicht, daß die Spannelemente auch bei maximaler Drahtspannung nicht außenseitig über den Mittelsteg des Spannrings hinausragen. Eine weitere Verbesserung der Stabilität kann dadurch erreicht werden, daß der Stellring neben dem sich radial nach außen forterstreckenden Mittelsteg einen radial innenseitig in der vom Mittelsteg aufgespannten Ebene verlaufenden Ringflansch aufweist.

Bei der zuletzt genannten Ausgestaltung kann eine besonders einfache Verdrehsicherung der Spannelemente dadurch erreicht werden, daß diese auf der zum Zentrum des Stellrings hinweisenden Seite einen hammerkopfartigen Ansatz besitzen, der mit wenigstens einer

-14-

-14-

an dem innenseitigen Ringflansch des Stellrings anliegenden Flachseite versehen ist, die beim Spannen des Spannelementes mittels des Einstellgewindes ein Mitdrehen des Spannelementes und damit eine Verdrillung des mit dem Spannelement verbundenen Drahtes wirksam verhindert.

Eine andere wichtige Ausgestaltung der Erfindung sieht vor, daß die Spannelemente zum Verbinden der Enden der Spanndrähte mit einem Stellring als auf letzteren aufschiebbare und mittels Klemmung festlegbare Halterungen mit U-förmigen Teilen ausgebildet sind sowie sich an Widerlagern dieser Halterungen abstützende und nachspannbare Spannhülsen aufweisen, in denen die Spanndrahtenden mittels Klemmung fest, jedoch lösbar verankert sind. Bei dieser Ausgestaltung können insbesondere planparallel ausgebildete Stellringe verwendet werden und die Halterungen der Spannelemente in Umfangsrichtung der Stellringe auf diesen stufenlos verschiebbar sowie infolge Klemmung mit dem jeweiligen Stellring in jeder Einstellage arretierbar sein, was sich im Interesse von Korrekturmanövern als nützlich und vorteilhaft erwiesen hat.

Eine Weiterbildung dieser Ausgestaltung sieht vor, daß die Spannelemente radial geführte und mittels sich an Widerlagern abstützender Spannmuttern in Spannlage bringbare Spannhülsen aufweisen, in denen infolge Klemmung die Enden von Spanndrähten festlegbar sind. Die Spannhülsen sind dabei zweckmäßigerweise so ausgebildet, daß sich von einem Stirnende aus jeweils eine Gewindespindel mit einer auf dieser aufgeschraubten Spannmutter fort- und durch eine Ausnehmung in dem Widerlager der die Spannhülse auf-

-15-

nehmenden Halterung längsbeweglich hindurcherstreckt,
wobei der mit seinem Ende in der Spannhülse festgelegte
Spanndraht in einer Mittelbohrung der Gewindespindel
aufgenommen sowie in der Spannhülse festgelegt ist
und sich die auf die Gewindespindel aufgeschraubte
Spannmutter radial außenseitig an dem Widerlager
abstützt, so daß durch Drehung der Spannmutter die
bei bestimmungsgemäßer Verwendung erforderliche Längsspannung auf einen mit seinem Ende an der Spannhülse
festgelegten Spanndraht aufgebracht werden kann und
in einfacher Weise auch eine Nachkorrektur der Drahtspannung gelingt. Dabei kann sich von der die Gewindespindel aufnehmenden Ausnehmung ein das Widerlager
seitlich durchbrechender Schlitz forterstrecken,
dessen Breite größer als der Spanndrahtdurchmesser,
aber kleiner als der Durchmesser der Gewindespindel
bemessen ist.

Eine ebenfalls vorteilhafte Weiterbildung dieser Ausgestaltung sieht vor, daß innerhalb einer sich von der
von der Gewindespindel entfernten Stirnseite aus in
ein Mittelteil der Spannhülse hineinerstreckenden
Bohrung, die in einer kegeligen Spannfläche endet, eine
spannfutterartige Klemmhülse mit durch Längsschlitze
voneinander getrennten Klemmfingern aufgenommen ist, die
eine ein eingeführtes Spanndrahtende aufnehmende Längsausnehmung aufweist und mittels einer in ein Innengewinde
der Bohrung eingeschraubten Spannschraube mit ihren
Spannfingern infolge deren Zusammenwirkens mit der
kegeligen Spannfläche das Spanndrahtende durch Klemmung
festlegt.

Gemäß einer wichtigen Weiterbildung dieser Ausgestaltung kann auch beidseitig des radial von innen auf einen Stellring aufschiebbaren und an diesem befestigbaren U-förmigen Teils eines Spannelements eine Radialführung mit einer in dieser aufgenommenen und jeweils an einem Widerlager abgestützten Spannhülse angeordnet sein, so daß es mittels eines derartigen Spannelementes gelingt, zwei Spanndrähte zu verankern, die sich auf gegenüberliegenden Seiten eines Stellringes im wesentlichen in einer senkrecht zur Stellringebene verlaufenden Ebene erstrecken. Die Anordnung kann auch so getroffen sein, daß die auf gegenüberliegenden Seiten eines Stellringes angeordneten Führungen für je eine Spannhülse divergierend oder konvergierend zueinander verlaufen.

Durch die paarige Anordnung jeweils sich in einer im wesentlichen senkrecht zur Stellringebene verlaufenden Ebene erstreckende Spanndrähte kann eine außerordentliche Stabilität der Fixation eines mittels derartiger Spanndrähte festgelegten Knochenabschnittes erreicht werden, und zwar insbesondere dann, wenn die mittels einer Spannvorrichtung festgelegten Spanndrähte zwischen der Spannvorrichtung und dem Knochenabschnitt divergierend oder konvergierend verlaufen.

Anhand der beigefügten Zeichnung sollen nachstehend einige Ausführungsformen der Erfindung erläutert werden. In schematischen Ansichten zeigen:

Fig. 1 eine Draufsicht auf einen Stellring mit einander kreuzenden Spanndrähten, die sich im Abstand voneinander in der Stellringebene erstrecken und einen nur angedeuteten Röhrenknochen durchdringen,

Fig. 2    einen Schnitt gemäß der Schnittlinie II-II in

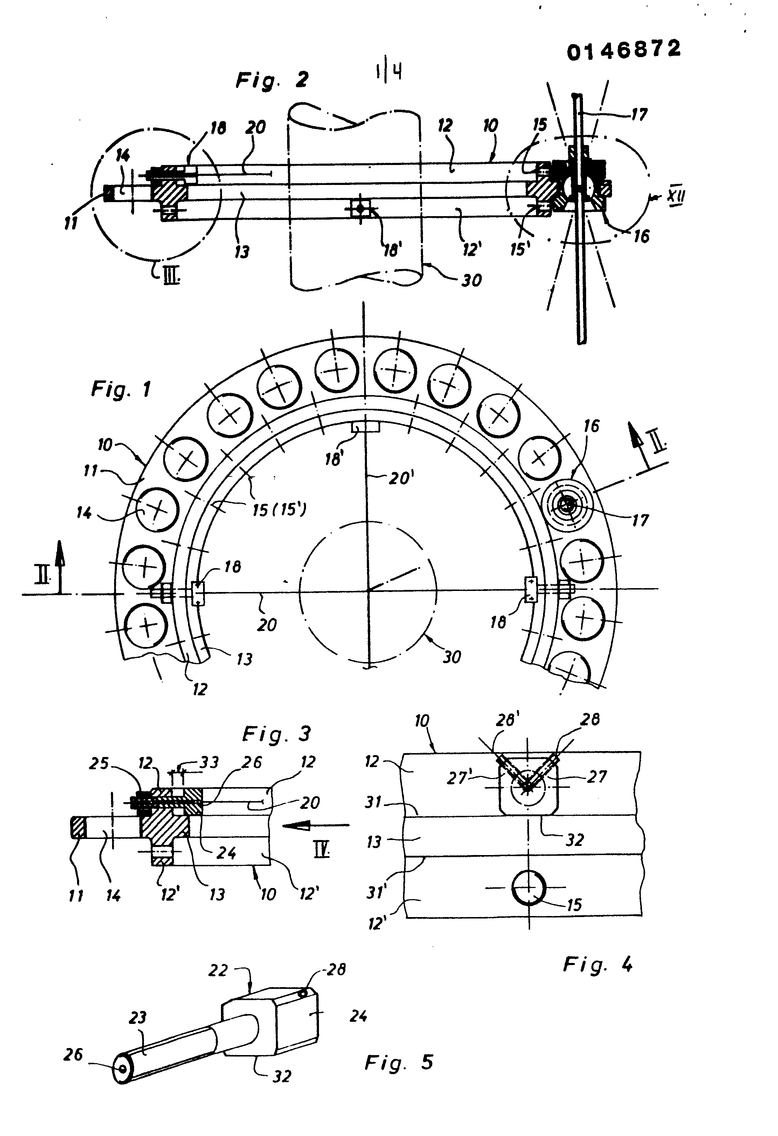

Fig. 1 durch den Stellring mit einer mittels
eines Verbindungselements angeschlossenen
Stange zum Verbinden des Stellrings mit einem
anderen, jenseits einer Bruchstelle des angedeuteten
Röhrenknochens zu plazierenden Stellring,

Fig. 3    in einer vergrößerten Ausschnittansicht gemäß
III aus Fig. 2 die Ausbildung eines Spannelementes in Form einer Spannhülse, die in
einer einen Flanschteil des Stellrings durchbrechenden Radialbohrung längsbeweglich aufgenommen und an der ein Ende eines Spanndrahtes
festgelegt ist,

Fig. 4    eine stark vergrößerte Ansicht eines Stellringausschnittes mit Spannhülse gemäß Pfeil IV in
Fig. 3,

Fig. 5    eine Spannhülse ohne Spannmutter für sich allein
in einer stark vergrößerten perspektivischen
Ansicht,

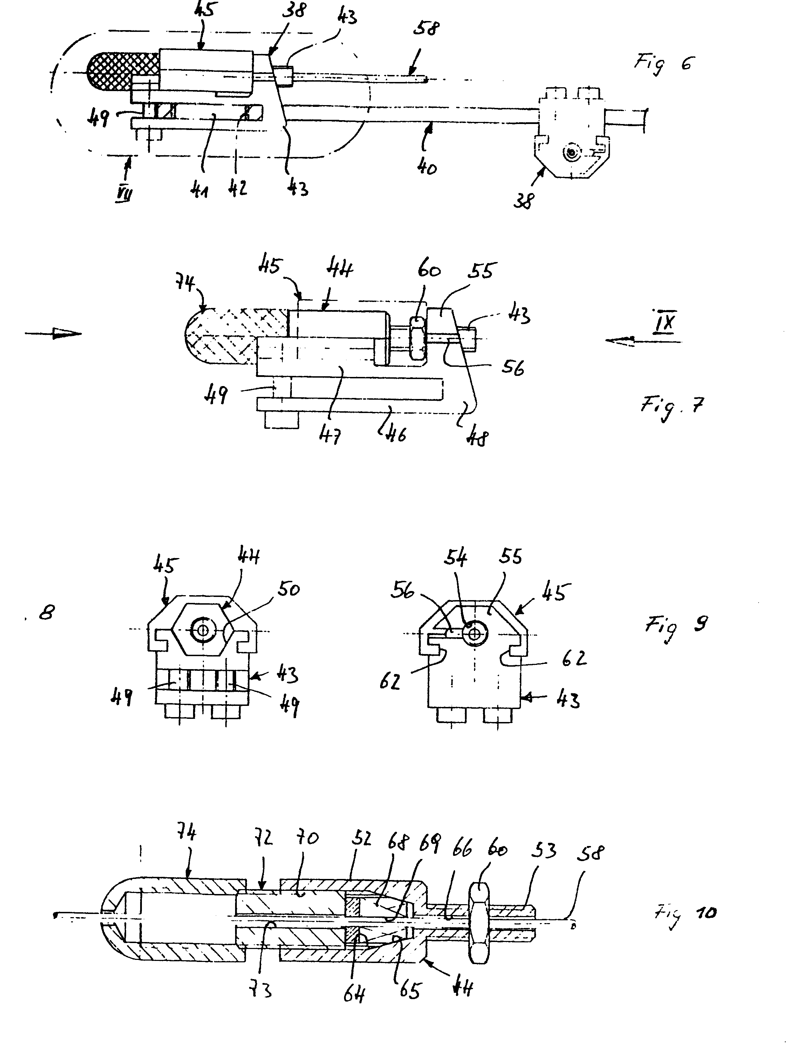

Fig. 6    in einer Schnittansicht ähnlich Fig. 2 eine andere
Ausführungsform eines Stellrings mit auf diesem
angeordneten, zu dem Spannelement nach Fig. 3
alternativ ausgebildeten Spannelementen für
die Spanndrähte,

Fig.7    in einer vergrößerten Ausschnittansicht gemäß
VII aus Fig. 6 ein Spannelement für sich allein
mit einer nur strichpunktiert angedeuteten
Abdeckung,

Fig. 8    eine stirnseitige Ansicht des Spannelementes
mit Blick gemäß Pfeil VIII in Fig. 7, jedoch
mit aufgesetzter Abdeckung,

Fig.9 ebenfalls eine stirnseitige Ansicht des Spannelementes mit Blick gemäß Pfeil IX in Fig.7,
ebenfalls mit aufgesetzter Abdeckung,

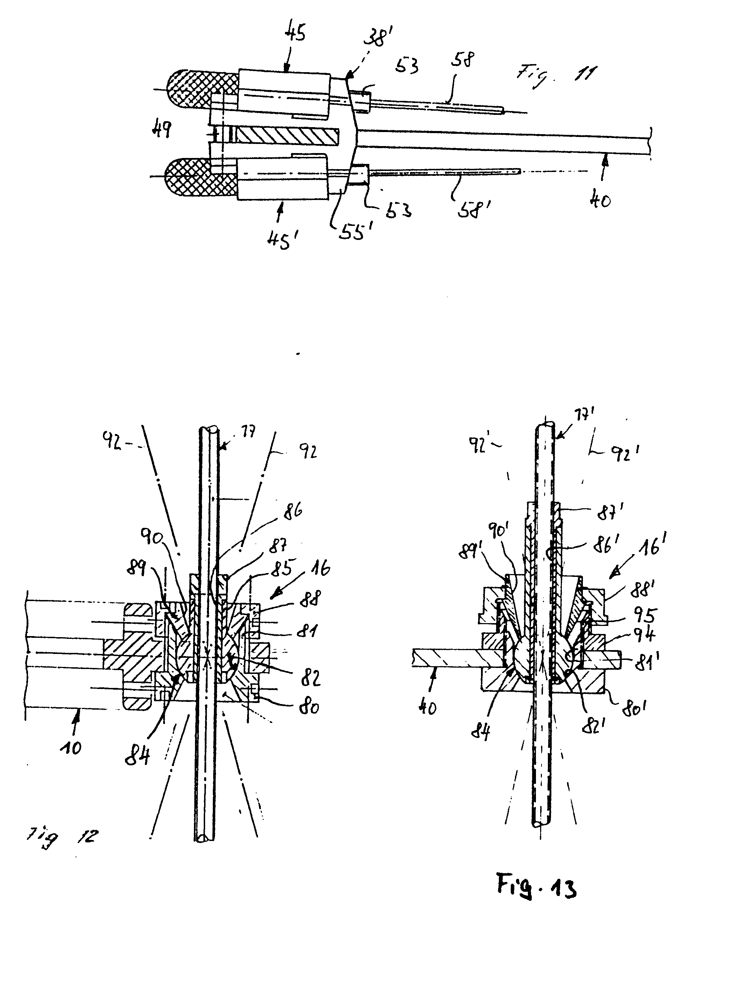

Fig.10 in einer nochmals gegenüber den Fig. 7 bis 9
vergrößerten Darstellung in einer teilweisen
Längsschnittansicht eine Spannhülse eines
Spannelements mit einer in dieser Spannhülse
aufgenommenen Klemmhülse,

Fig.11 in einer Ansicht wie in Fig. 7 die Ausbildung
eines Spannelementes zur Aufnahme und Halterung
je eines Spanndrahtes, die sich in einer Ebene
im wesentlichen senkrecht zur Stellringebene
und jeweils auf einer Stellringseite erstrecken,

Fig.12 das in Fig. 2 veranschaulichte Befestigungselement
in einer vergrößerten Ausschnittansicht gemäß
XII aus Fig. 2,

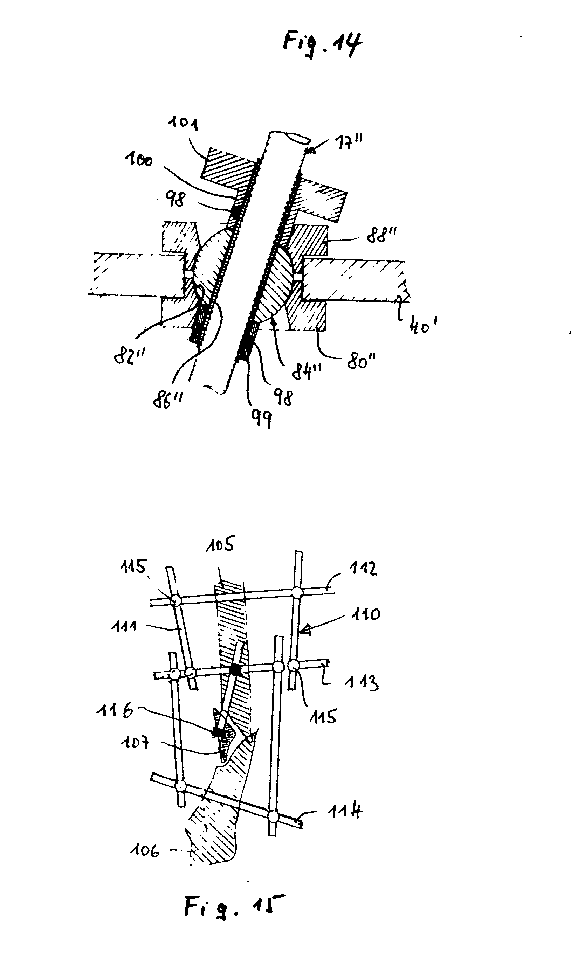

Fig. 13 in einer Ansicht wie in Fig. 12 eine alternative
Ausbildung eines Befestigungselements,

Fig. 14 eine zu den Befestigungselementen nach den
Fig. 12 und 13 alternative Ausbildung eines
Befestigungselements und Fig. 15 ein Montagebeispiel eines erfindungsgemäßen
Fixateurs bei einem Knochenbruchstück.

Der in seiner Gesamtheit mit 10 bezeichnete Stellring
nach den Fig. 1 und 2 hat einen T-förmigen Querschnitt mit einem sich radial nach außen forterstreckenden Mittelsteg 11, je einem sich auf jeweils einer Seite des Mittelstegs senkrecht zur Ringebene erstreckenden Flanschteil 12, 12' und mit einem inneren Ringflansch 13, der auf der zum Ringzentrum hinweisenden Seite der Flanschteile in der vom Mittelsteg aufgespannten Ebene umläuft. Der Mittelsteg 11 ist von axial gerichteten und in Umfangsrichtung in untereinander gleichen Abständen angeordneten Ausnehmungen 14 durchbrochen, während sich durch die Flanschteile 12, 12' jeweils in axialen Abständen von dem Mittelsteg 11 bzw. dem inneren Ringflansch 13 unter ebenfalls in Umfangsrichtung gleichen Abständen voneinander angeordnete Radialbohrungen 15, 15' hindurcherstrecken, die in Fig. 1 nur durch Mittellinien angedeutet, in den Ansichten der Flanschteile in Fig. 2 aber nicht dargestellt sind.

Die Ausnehmungen 14 im Mittelsteg 11 des Stellringes sind mit Innengewinde versehen und dienen der Aufnahme von Befestigungselementen 16, durch die sich Stangen 17 hindurcherstrecken. Diese Stangen 17 dienen zum Verbinden zweier Stellringe, die sich in voneinander entfernten, nicht notwendig parallel zueinander verlaufenden Ebenen erstrecken. Die Radialbohrungen 15, 15' in den Flanschteilen 12, 12' hingegen dienen der radial bewegbaren Aufnahme von Spannelementen 18, 18' zum Festlegen der Enden von Spanndrähten 20, 20' am Stellring.

Jedes der Spannelemente besteht aus einer Spannhülse 22 mit einer Gewindespindel 23 und einem hammerkopfartigen

-20-

Ansatz 24 an einem Spindelende sowie einer auf der Gewindespindel aufgenommenen Spannmutter 25. Durch die Spannhülse erstreckt sich eine zentrale Mittelbohrung 26 hindurch und in dieser Mittelbohrung münden zwei rechtwinklig zu letzterer verlaufende Gewindebohrungen 27, 27' mit Klemmschrauben 28, 28' aus, die in dem hammerkopfartigen Ansatz 24 etwa rechtwinklig zueinander und in Axialrichtung der Mittelbohrung 26 im Abstand voneinander angeordnet sind.

Bei bestimmungsgemäßer Verwendung der Vorrichtung werden beidseitig im Abstand von einer Bruchstelle die einzurichtenden Knochenabschnitte von in der Regel kreuzweise zueinander verlaufenden Spanndrähten 20, 20' durchdrungen, die mit ihren Enden an ihrerseits mittels der Stangen 17 miteinander verbundenen Stellringen 10 befestigt sind. Die an einem Stellring befestigten Spanndrähte 20, 20' erstrecken sich in Parallelebenen des Stellrings im Abstand der in den Flanschteilen 12, 12' angeordneten Radialbohrungen 15, 15' voneinander. In den Fig. 1 und 2 ist ein von den Spanndrähten durchdrungener Knochenabschnitt 30 angedeutet.

Nachdem die Spanndrähte 20, 20' im Abstand von einer Bruchstelle im Knochenabschnitt 30 plaziert sind, wird jedes Ende eines Spanndrahtes von dem hammerkopfartigen Ansatz 24 aus in die Mittelbohrung 26 einer Spannhülse 22 eingeführt und durch Festziehen der Klemmschrauben 28, 28' mit der Spannhülse fest verbunden, und zwar in der Weise, daß die Mittelteile der dann

-21-

842312

—21—

jeweils nach außen weisenden Gewindespindeln 23 der
Spannhülsen eines Spanndrahtes 20, 20' sich etwa
in einem dem Durchmesser der die Radialbohrungen
15, 15' aufweisenden Flanschteile 12, 12' des Stellrings 10 entsprechenden Abstand voneinasnder erstrecken
und die hammerkopfartigen Abschnitte 24 einen Abstand
voneinander haben, der kleiner ist als die lichte
Weite des Ringflansch 13, jedoch größer als der Innendurchmesser der Flanschteile 12, 12'. Danach wird
der Stellring rontiert, indem die Gewindespindeln
beispielsweise der mit dem Spanndraht 20 verbundenen
Spannhülsen in einander gegenüberliegende Radialbohrungen 15 im Flanschteil 12, hingegen die Gewindespindeln der mit dem Spanndraht 20' in entsprechender Weise verbundenen Spannhülsen in Radialbohrungen
15' des Flanschteils 12' eingeführt und anschließend
auf die sich radial außenseitig von den Flanschteilen
forterstreckenden Abschnitte der Gewindespindeln
23 Spannmuttern 25 aufgeschraubt werden. Die hammerkopfartigen Ansätze 24 der Spannhülsen 22 haben eine
dann an der jeweils benachbarten Ober- oder Unterseite
31, 31' des Ringflanschs 13 anliegende und dadurch
die Spannhülsen gegen Verdrehung sichernde Flachseite
32. Durch Anziehen der Spannmuttern 25 werden dann
die Spanndrähte 20, 20' im Rahmen des Spiels 33
zwischen den hammerkopfartigen Ansätzen 24 und den
Flanschteilen 12, 12' des Stellrings 10 gespannt
und, sofern dies nicht bereits geschehen ist, anschließend die beidseitig einer Bruchstelle angeordneten Stellringe mittels der Stangen 17 miteinander
verbunden.

-22-

Anstelle eines im Querschnitt T-förmigen Stellringes kommt in Verbindung mit den in den Fig. 6 bis 11 veranschaulichten Spannelementen 38 ein Stellring 40 ohne sich rechtwinklig zur Ringebene erstreckenden Flanschteilen zum Einsatz, der ebenso wie der Stellring 10 nach Fig. 2 mit in Umfangsrichtung in untereinander gleichen Abständen voneinander angeordneten Ausnehmungen 44 versehen ist, bei denen es sich um mit Innengewinde 42 versehene Lochungen handelt.

Die Spannelemente 38 nach den Fig. 6 bis 10 bestehen aus einer auf dem Stellring 40 festlegbaren Halterung 43, einer Spannhülse 44 und einer Abdeckung 45. Die Halterung besitzt einen U-förmigen Klemmabschnitt zum Aufschieben auf den Stellring radial von innen, der in der aus Fig. 6 ersichtlichen Montagelage aus zwei radial außenseitig über den Stellring hinausragenden, den Flanschen 46, 47 und einem letztere verbindenden Steg 48 besteht. Gesichert ist die Halterung 43 auf dem Stellring 40 mittels Klemmschrauben 49, die sich radial außenseitig vom Stellring durch den einen Flansch 46 des U-förmigen Teils hindurcherstrecken und in Gewindelöcher des anderen Flanschs 47 eingeschraubt sind. Angesichts dieser Aufnahme der Halterungen auf dem Stellring gelingt es in einfacher Weise, die Spannelemente 38 nach dem Lösen der Klemmschrauben 49 in Umfangsrichtung des Stellringes stufenlos zu verschieben und infolge Klemmung des U-förmigen Teils mit dem Stellring in jeder Position wieder zu arretieren.

-23-

Auf der von dem U-förmigen Teil entfernten Seite des Flanschs 47 erstreckt sich in der Halterung eine -in der Montagelage- radial verlaufende Trapezführung 50, in der die in Fig. 10 für sich allein veranschaulichte Spannhülse44 aufgenommen ist. Ein Mittelteil 52 der Spannhülse 44 besitzt Sechskantquerschnitt und von einem Stirnende dieses Mittelteils erstreckt sich eine Gewindespindel 53 fort und durch eine mit der Trapezführung 50 fluchtende Ausnehmung 54 in einem Widerlager 55 der Halterung 43 hindurch, das sich rechtwinklig zu der Trapezführung und im Abstand von dieser etwa im Bereich des die beiden Flanschen 46, 47 der Halterung miteinander verbindenden Stegs 48 aus der Führungsebene erhebt. An die sich durch das Widerlager 55 hindurcherstreckende und die Gewindespindel 53 aufnehmende Ausnehmung 54 im Widerlager 55 schließt sich ein das Widerlager seitlich durchbrechender Schlitz 56 von einer solchen Breitenerstreckung an, daß durch diesen Schlitz ein Spanndraht 58,58' von der Seite aus in den Bereich der genannten Ausnehmung eingeführt werden kann. Mittels einer auf die Gewindespindel 53 aufgeschraubten Spannmutter 60 stützt sich die Spannhülse 44 am Widerlager 55 ab und ist relativ zu diesem einstellbar. Auf der von den Flanschen 46, 47 der Halterung entfernten Seite ist die Spannhülse 44 von einer ihrerseits in Parallellage zur Trapezführung verlaufenden Nutführungen 62 aufgenommenen und somit von der radial von außen zum Stellring aufschiebbaren Abdeckung 45 übergriffen.

-24-

-24-

Von der von der Gewindespindel 53 entfernten Stirnseite aus erstreckt sich in das Mittelteil 52 der Spannhülse 44 eine Bohrung 64 hinein, die in der Nähe der anderen Stirnseite in einer kegeligen Spannfläche 65 endet, von der sich eine mit der genannten Bohrung fluchtende Längsbohrung 66 durch die Gewindespindel hindurch, deren Querschnitt etwas größer als der Querschnitt der einzusetzenden Spanndrähte 58, aber sehr viel kleiner als die Bohrung 64 im Mittelteil 52 bemessen ist. In der Bohrung im Mittelteil ist eine spannfutterartige Klemmhülse 68 aufgenommen, die auf der an der kegeligen Spannfläche 65 des Mittelteils anliegenden Seite mit durch Längsschlitze 69 voneinader getrennten Klemmfinger versehen ist, deren Enden mit der kegeligen Spannfläche zusammenwirken. In ein Innengewinde 70 der sich im Mittelteil erstreckenden Bohrung 64 ist eine Spannschraube 72 eingeschraubt, die stirn-seitig an der Klemmhülse 68 angreift. Schließlich ist von der vom Mittelteil 52 entfernten Seite auf die Spannschraube 72, durch die sich eine mit der Längsbohrung 66 fluchtende Längsbohrung 73 hindurcherstreckt, eine mit einer Ränderlung versehene Schutzkappe 74 aufgeschraubt.

Auch bei dieser Ausführungsform werden die Enden der Spanndrähte, nachdem diese lagerichtig in einem Knochen plaziert sind, in die Längsbohrungen 66 der Gewindespindeln 53 der Spannhülsen 44 eingeführt, und zwar in der Weise, daß die Spanndrahtenden sich durch die Klemmhülse 68 hindurch und ggf. auch in die Längsbohrungen 73 in den Spannschrauben 72 hineinerstrecken. Durch Anziehen der Spannschrauben werden dann die Klemmfinger der Klemmhülsen 68 infolge ihres Zusammenwirkens mit den die Bohrungen 64 in den Mittelteilen 52 der Spannhülsen axial begrenzenden Spannflächen 65 radial verformt und dadurch die Spanndrahtenden in der bei Spannfuttern bekannten Weise infolge Klemmung axial festgelegt. Die erforderliche Drahtspannung wird dann durch Drehen der an dem Widerlager 55 der Halterung anliegenden und auf der Gewindespindel 53 aufgenommenen Spannmutter 60 aufgebracht. Beim Festlegen der Spanndrahtenden mittels radialer Klemmung durch die Klemmfinger der Klemmhülse 68 ist darauf zu achten, daß die Spannhülse 44 sich in bezug auf die auf dem Stellring 40 aufgenommene Halterung etwa in einer Fig. 7 entsprechenden Lage befindet, so daß im Rahmen des sich durch die Ausnehmung 54 im Widerlager 55 der Halterung hindurcherstreckenden Abschnittes der Gewindehülse 44 eine axiale Drahtspannung bewerkstelligt werden kann.

Bei der Festlegung der Spanndrahtenden kann auch in der Weise vorgegangen werden, daß zumindest ein Ende eines Spanndrahtes 58 unabhängig von der Aufnahme einer Spannhülse 44 in der zugeordneten Halterung 43 an der Spannhülse befestigt wird, und zwar in einem dem Abstande der radial nach außen weisenden Spannfläche des Widerlagers 55 der zugeordneten Halterung entsprechenden Abstande vom Zentrum eines von Spanndrähten durchdrungenen Knochens, worauf der entsprechende Stellring 40 mit der darauf montierten Halterung außermittig an den aus der Gewindehülse 53 austretenden Abschnitt des Spanndrahtes herangeführt und letzterer durch den sich seitwärts von der das Widerlager 55 durchdringenden Ausnehmung 54 erstreckenden Querschlitz 56 hindurch in die Ausnehmung im Widerlager eingeführt wird, worauf eine im wesentlichen zentrische Positionierung des Stellringes 40 und dabei ein Einschieben der Gewindespindel 53 in die Ausnehmung im Widerlager gelingt, wie dies insbesondere Fig. 7 zeigt. Bei dieser Montage wird das Sechseckquerschnitt aufweisende Mittelteil 52 in der sich radial erstreckenden Trapezführung der Halterung aufgenommen und durch nachträgliches Aufschieben der Abdeckung 45 in die letztere aufnehmenden Nutführungen 62 in seiner Lage gesichert.

Es ist ersichtlich, daß Spannelemente der vorstehend in Verbindung mit den Fig. 1 bis 10 beschriebenen Art eine äußerst einfache und schnelle Montage ermöglichen.

Das in Fig. 11 veranschaulichte Spannelement 38' unterscheidet sich im wesentlichen nur dadurch von dem Spannelement nach den Fig. 6 bis 10, daß beidseitig der mit ihrem U-förmigen Teil radial von innen auf einen Spannring 40 aufschiebbaren Halterung 43' je eine mittels einer in Nutführungen aufgenommenen Abdeckung 45, 45' gesicherte Spannhülse angeordnet ist, deren Gewindespindeln 53, 53' sich durch Ausnehmungen in den symmetrisch zu der Stellringebene angeordneten Widerlagern 55, 55' hindurcherstrecken. Mithin ermöglicht dieses Spannelement die Anbringung je eines Spanndrahtes 58, 58' auf jeder Seite des Stellringes, wobei die einander zugeordneten Spanndrähte sich im wesentlichen in einer Ebene senkrecht zur Stellringebene erstrecken.

Der besondere Vorteil dieser Spannelementausbildung besteht darin, daß die beiden sich in einer Ebene im wesentlichen rechtwinklig zur Stellringebene erstreckenden Spanndrähte konvergierend oder auch divergierend zueinander verlaufen können, was bei Aufbringung einer entsprechenden Drahtspannung zu einer bei Einfachdrähten nicht möglichen Stabilisierung von Knochenabschnitten bzw. Knochenfragmenten führt. Insbesondere gilt dies, wenn zwei Drahtpaare rechtwinklig zueinander verlaufen, wie dies für Einfachdrähte oben in Verbindung mit Fig. 2 erläutert worden ist, wobei die Spanndrähte des einen Drahtpaares beispielsweise divergierend zueinander, die Spanndrähte des anderen Drahtpaares konverierend zueinander verlaufen können.

Bei dem in Fig. 12 in gegenüber Fig. 2 vergrößerter Darstellung veranschaulichten Befestigungselement 16 handelt es sich um ein Kugelgelenk mit einer Lagerschale 80, die mittels eines Gewindeabschnittes 81 in ein Innengewinde in einer den Stellring durchdringenden Ausnehmung 14 eingeschraubt ist. Der Gewindeabschnitt 81 erstreckt sich in dieser Einschraublage auf der von der Lagerschale entfernten Seite über den Stellring 10 hinaus. Innerhalb der Lagerschale ist in einer sphärisch konfigurierten Lagerfläche 82 eine Lagerkugel 84 aufgenommen, von der sich ein Hülsenteil 85 forterstreckt, das mit der die Lagerkugel durchdringenden Längsbohrung fluchtet. In der sich durch die Lagerkugel und das Hülsenteil erstreckenden Längsbohrung ist eine Gewindehülse 86 drehbar, aber axialfest gelagert, die an dem von der Lagerkugel

- 28-

entfernten Ende mit einer Schlüsselansatzfläche 87 zum Aufbringen von Drehmomenten versehen ist und in die eine Gewindestange 17 zum Verbinden des das Befestigungselement 16 aufnehmenden Stellringes mit einem anderen, mit einem gleichartigen Befestigungselement ausgerüsteten Stellring eingeschraubt ist. Diese Gewindestange erstreckt sich beidseitig von der Gewindehülse 86 fort und durch eine sich in der Lagerschale 80 von der sphärisch konfigurierten Lagerfläche 82 aus konisch erweiternde Mittelausnehmung hindurch. Auf den über den Stellring hinausragenden Teil des Gewindeabschnittes 81 ist ein in der Art einer Überwurfmutter ausgebildeter Klemmring 88 mit einer Klemmhülse 89 aufgeschraubt, wobei die Klemmhülse einen sich zur Lagerkugel 84 hin verengenden Innenkonus 90 aufweist, der innerhalb eines vorbestimmten Winkelbereichs allseitige Schwenkbewegungen der Lagerkugel 84 gegenüber der Lagerschale 80 und damit der Gewindestange 17 gegenüber dem Stellring 10 ermöglicht, wie die mit 92 bezeichneten strichpunktierten Linien andeuten. Die Klemmhülse 89 greift mit ihrem vom Klemmring 88 entfernten Ende an der Lagerkugel 84 an und arretiert diese infolge Klemmung, wenn der Klemmring 88 angezogen ist. Bei gelöstem Klemmring 88 hingegen ist die Lagerkugel und damit die sich durch diese hindurcherstreckende Gewindestange 17 innerhalb des durch die strichpunktierten Linien 92 angegebenen Bereichs beliebig verschwenkbar.

Bei der Ausführungsform nach Fig. 13 sind für gleiche Teile wie in Fig. 12 die gleichen Bezugszeichen verwendet worden, zur Unterscheidung aber durch einen Strich gekennzeichnet.

Das Befestigungselement 16' ist in einer mit einem Innengewinde 42 versehenen Ausnehmung 41 des in Fig. 6 angedeuteten Stellrings 40 aufgenommen, in dem ein Gewindeabschnitt 81' der sphärisch konfigurierte Lagerflächen 82' aufweisenden Lagerschale 80' in das Innengewinde der Ausnehmung eingeschraubt ist. Zur Sicherung der Lagerschale in dieser Montagelage ist auf den sich auf der von der Lagerschale entfernten Seite des Stellringes forterstreckenden Teil des Gewindeabschnittes 81' eine Kontermutter 94 aufgeschraubt, die ebenfalls einen sich auf der vom Stellring abgewandten Seite forterstreckenden Gewindeabschnitt 95 besitzt, auf den der Klemmring 88' aufgeschraubt ist. Der Klemmring ist wiederum mit einer Klemmhülse 89' ausgerüstet, die einen sich zur Lagerkugel 84' hin verengenden Innenkonus 90' aufweist und mit ihrem vom Klemmring entfernten Ende an der Lagerkugel 84' angreift. Wie bei der Ausführungsform nach Fig. 12 gelingt somit infolge Anziehens des auf dem Gewindeabschnitt 95 der Kontermutter 94 aufgenommenen Klemmrings eine Arretierung der Gewindestange 17 in der jeweiligen Einstellage, hingegen bei gelöstem Klemmring 88' eine beliebige Verschwenkung der Lagerkugel mit der Gewindestange innerhalb des durch die strichpunktierten Linien 92' angedeuteten Verschwenkbereichs.

Im Unterschied zu der Ausführungsform nach Fig. 12 ist jedoch bei dieser Ausführungsform des Befestigungselementes die Klemmhülse 89' nicht dreh- und axialfest mit dem Klemmring 88' verbunden, sondern axialfest,

aber drehbar am Klemmring gelagert. Dabei stützt sich ein radial auskragender Ringbund der Klemmhülse an einen inneren Ringflansch des über-wurfmutterartig ausgebildeten Klemmringes 88' ab und innerhalb einer zentralen Mittelausnehmung des Klemmringes erstreckt sich durch diesen ein Abschnitt der Klemmhülse fort, der auf der von der genannten Ringschulter entfernten Seite des Klemmrings mittels eines nur angedeuteten Sprengrings gesichert ist. Angesichts dieser Ausbildung gelingt ein Arretieren der Lagerkugel in der jeweiligen Einstellage bei nicht mitdrehender Klemmhülse, wobei die zusammenwirkenden Kontaktflächen im Bereich des Ringflanschs des Klemmringes 88' und der Schulter der Klemmhülse 89' so ausgebildet sind, daß nur geringe Lagerreibung auftritt.

Im übrigen unterscheidet sich der Aufbau des Befestigungselements 16' nicht von dem des Befestigungselements 16 nach Fig. 12.

Bei der Ausführungsform des Befestigungselementes 16" nach Fig. 14 ist die Gewindestange 17" zum Verbinden benachbarter Stellringe ebenfalls in einer Gewindehülse 86" aufgenommen, die sich durch eine Mittelausnehmung in der Lagerkugel 84" hindurcherstreckt. Auf sich beidseitig über die Lagerkugel hinauserstreckende Abschnitte der Gewindehülse 86" sind mittels Madenschrauben 98 gesicherte Hülsenteile 99, 100 dreh- und axialfest aufgesetzt, womit die Gewindehülse 86" innerhalb der sich durch die Lagerkugel hindurcherstrecken den Ausnehmung axialfest, jedoch drehbar aufgenommen ist. Das eine axial- und drehfest mit der Gewindehülse
85" verbundene Hülsenteil 100 ist mit einem Rändelrand
101 versehen, mittels dessen eine Drehung der Gewindehülse und damit eine Axialverschiebung der in letztere
eingeschraubten Gewindestange 17" gegenüber der Gewindehülse 86" und damit gegenüber der Lagerkugel 84"
gelingt. Die Lagerkugel ist zwischen einer mit
sphärischen Lagerflächen versehenen Lagerschale 80"
und einem ebenfalls eine sphärisch konfigurerite
Kontaktfläche aufweisenden Klemmring 88" aufgenommen,
die in ein Innengewinde einer den Stellring senkrecht
zur Ringebene durchbrechenden Ausnehmung eingeschraubt
sind.

Auch dieses Befestigungselement 16" ermöglicht, ebenso
wie die Ausführungsformen nach den Fig. 12 und 13
eine stufenlose Winkeleinstellung der Gewindestange 17"
gegenüber der Ebene des das Befestigungselement aufnehmenden Stellringes innerhalb der konstruktiv vorgegebenen Grenzen, desgleichen eine Arretierung in
jeder einstellbaren Winkellage, indem der Klemmring 88"
los- und festdrehbar ist, ferner aber auch unabhängig
von der Winkeleinstellung eine Abstandseinstellung
benachbarter Stellringe infolge Axialverschiebung
der Gewindestange 17", was durch Drehung der Gewindehülse 86" mittels Betätigung des mit dem Rändelrand 101 versehenen Hülsenteils 100 gelingt.

Bei dem Montagebeispiel nach Fig. 15 handelt es sich
um die Fixation zweier Knochenabschnitte 105, 106
und eines Knochenfragments 107 im Bereich einer Bruchstelle mittels eines erfindungsgemäßen Fixateurs 110.
Der Fixateur umfaßt drei jeweils durch Gewindestangen
111 miteinander verbundene Stellringe 112, 113 und 114, wobei die Stellringe 112, 113 der Fixation des einen Knochenabschnitts 105 und der dritte Stellring 114 der Fixation des anderen Knochenabschnittes 106 dienen. In den Kreuzungsstellen 115 zwischen den Stangen 111 und Stellringen 112, 113, 114 befinden sich Befestigungselemente der oben in Verbindung mit den Fig. 12 bis 14 beschriebenen Art, die sowohl ein Verschwenken der Stangen gegenüber der jeweiligen Stellringebene und eine Fixation in der jeweiligen Einstellage als auch unabhängig von der Winkeleinstellung der Stangen 111 gegenüber der jeweiligen Stellringebene eine Längenänderung der sich zwischen benachbarten Stellringen erstreckenden Abschnitte der Stangen und damit eine Einstellung der Stellringe in nicht parallelen Ebenen ermöglichen. Die Fixation der Knochenabschnitte 105, 106 ist mittels nicht weiter dargestellter Spanndrähte verwirklicht, die in den jeweiligen Stellringebenen kreuzweise zueinander verlaufen und deren Enden jeweils auf gegenüberliegenden Seiten an den Stellringen festgelegt sind. An dem mittleren Stellring 113 des Fixateurs ist eine weitere Funktionseinheit aus parallel zueinander verlaufenden und in Befestigungselementen an diesem Stellring gelagerten Stangen 111 angeordnet, deren Enden mit einer Bohrdrahtfixation 116 zur Erfassung und Positionierung des abseits liegenden kleineren Knochenfragmentes 106 versehen sind.

842312

Anmelder:    Schewior, Thomas, Dr. med.
             Lessingstraße 1


             D-6903 Neckargemünd



Patentansprüche:
==================


1. Vorrichtung zum Einrichten von Knochenabschnitten und/oder Knochenfragmenten, bestehend aus durch Stangen und Befestigungselemente in ein- und verstellbaren Abständen miteinander verbindbaren Stellringen und aus mit letzteren unter Längsspannung verbindbaren, sich in den von den Stellringen aufgespannten Ebenen erstreckenden undmit ihren Enden mittels Halterungen an voneinander entfernten Teilen jeweils eines Stellringes befestigbaren Spanndrähten zur Fixation der Knochenabschnitte oder -fragmente,
dadurch gekennzeichnet,
daß die Stangen (17, 17', 17'') mittels der Befestigungselemente (16, 16', 16'') innerhalb vorbestimmter Grenzen allseitig schwenkbar an den Stellringen (10,40) gelagert und in jeder Schwenkstellung arretierbar sind, und daß die Abstände zwischen benachbarten Stellringen unabhängig von den eingestellten Schwenklagen der Stangen einstellbar sind.


-2-

2. Vorrichtung nach Anspruch 1, dadurch gekennzeichnet, daß die Stellringe (10,40) mit sich senkrecht zu den von ihnen aufgespannten Ebenen erstreckenden und über den Umfang verteilt angeordneten Ausnehmungen (14, 41) für die austauschbare Aufnahme der Befestigungselemente (16, 16', 16'') versehen sind, und daß die in diesen Ausnehmungen fest, jedoch lösbar montierbaren Befestigungselemente sphärisch konfigurierte, in jeder Einstellage stufenlos arretierbare Schwenklagerungen sowie stufenlos verstellbare Längslagerungen für die Stangen aufweisen.

3. Vorrichtung nach Anspruch 2, dadurch gekennzeichnet, daß jede Schwenklagerung eine in einer spährische Lagerflächen (82, 82', 82") aufweisenden Lagerschale (80, 80', 80") aufgenommene Lagerkugel (84, 84', 84"), durch die sich eine Stange (17, 17',17") hindurcherstreckt, sowie eine mittels Schraubgewinde mit der Lagerkugel verspannbare und letztere in der Spannlage arretierende Klemmeinrichtung besitzt.

4. Vorrichtung nach Anspruch 3, dadurch gekennzeichnet, daß die Lagerschale (80, 80',80") mittels eines hülsenartigen Abschnittes (81, 81',81"), der in eine das Befestigungselement aufnehmende Ausnehmung (14, 41) im Stellring hineinragt, in letzteren fest, jedoch lösbar verankert ist.

5. Vorrichtung nach Anspruch 4, dadurch gekennzeichnet, daß der hülsenartige Abschnitt der Lagerschale (80, 80',80") ein mit Außengewinde versehener Gewindeabschnitt (81, 81',81") ist.

6. Vorrichtung nach Anspruch 5, dadurch gekennzeichnet, daß die Lagerschale (80, 80',80") mittels des Gewindeabschnittes (81,81',81") in ein Innengewinde der das Befestigungselement aufnehmenden Ausnehmung (14, 41) im Stellring eingeschraubt ist.

7. Vorrichtung nach einem der Ansprüche 3 bis 6, dadurch gekennzeichnet, daß die Klemmeinrichtung zum Arretieren der Lagerkugel (84, 84',84") einen mittels eines Schraubgewindes relativ zu der die Lagerkugel aufnehmenden Lagerschale (80,80', 80") einstellbaren und in Abhängigkeit von seiner Einstellung an der Lagerkugel angreifenden Klemmring (88, 88',88") umfaßt.

8. Vorrichtung nach Anspruch 7, dadurch gekennzeichnet, daß der Klemmring (88, 88',88") mit seinem Schraubgewinde in ein Innengewinde der die Lagerschale (80,80',80") aufnehmenden Ausnehmung im Stellring eingeschraubt ist.

9. Vorrichtung nach einem der Ansprüche 4 bis 8, dadurch gekennzeichnet, daß der in die Ausnehmung im Stellring hineinragende Abschnitt (81, 81') der Lagerschale (80,80') eine Länge größer als die Dicke des Stellrings aufweist und daß wenigstens der sich auf der von der Lagerschale entfernten Seite erstreckende Teil dieses Abschnittes als Gewindeabschnitt ausgebildet ist.

10. Vorrichtung nach Anspruch 9, dadurch gekennzeichnet, daß der Klemmring (88) auf den sich durch die Ausnehmung im Stellring hindurcherstreckenden
Teil des Gewindeabschnittes (81) der Lagerschale
(80) aufgeschraubt ist.

11. Vorrichtung nach Anspruch 9, dadurch gekennzeichnet, daß auf dem sich durch die Ausnehmung im
Stellring hindurcherstreckenden Teil des Gewindeabschnittes (81) der Lagerschale (80') eine letztere
in der Einschraublage sichernde Kontermutter (94)
aufgeschraubt ist.

12. Vorrichtung nach Anspruch 11, dadurch gekennzeichnet, daß der Gewindeabschnitt (81') der Lagerschale (80') in die Ausnehmung im Stellring einsteckbar
und durch formschlüssig mit der Ausnehmung zusammenwirkende Mittel gegen Drehung gesichert ist.

13. Vorrichtung nach Anspruch 11 oder 12, dadurch
gekennzeichnet, daß die Kontermutter (94) mit einem
sich auf der von der Lagerschale (80') entfernten
Seite forterstreckenden Gewindeabschnitt (95) versehen
und auf letzteren der Klemmring (88') aufgeschraubt
ist.

14. Vorrichtung nach Anspruch 8 oder 13, dadurch
gekennzeichnet, daß zwischen dem Klemmring (88,88')
und der Lagerkugel (84,84') eine mittels des Klemmringes
gegenüber der Lagerkugel verspannbare Klemmhülse (89,
89') mit einem sich von der Lagerkugel aus erweiternden
und innerhalb eines vorbestimmten Winkelbereichs
Schwenkbewegungen der Lagerkugel mit der sich durch
diese hindurcherstreckenden Stange (17, 17') zulassenden Innenkonus (90,90') angeordnet ist.

15. Vorrichtung nach Anspruch 14, dadurch gekennzeichnet, daß die Klemmhülse (89') mit dem sich von der Lagerkugel (84') aus öffnenden Innenkonus (90') mit dem Klemmring (88') drehbar, aber axialfest verbunden ist.

16. Vorrichtung nach einem der Ansprüche 3 bis 15, dadurch gekennzeichnet, daß die sich durch die Lagerkugel (84, 84', 84") hindurcherstreckende Stange (17, 17', 17") als Gewindestange ausgebildet und in einer in der Lagerkugel drehbar, aber axialfest gelagerten Gewindehülse (86, 86',86") aufgenommen ist.

17. Vorrichtung nach Anspruch 16, dadurch gekennzeichnet, daß die in der Lagerkugel (84, 84', 84") drehbar, aber axialfest aufgenommene Gewindehülse (86, 86', 86") im Bereich eines sich in Axialrichtung über die Klemmeinrichtung hinauserstreckenden Teils mit einem Rändelring (101) bzw. einer Schlüsselansatzfläche (87,87') zum Aufbringen einer Drehbewegung um ihre Längsachse versehen ist.

18. Vorrichtung nach Anspruch 16 oder 17, dadurch gekennzeichnet, daß die sich zwischen benachbarten Stellringen erstreckenden Gewindestangen (17, 17', 17") mit Ansatzflächen zur Aufbringung von Drehmomenten bzw. zum Festhalten gegen Drehung versehen sind.

19. Vorrichtung zum Einrichten von Knochenabschnitten und/oder Knochenfragmenten, bestehend aus durch Stangen und Befestigungsmittel in ein-und verstellbaren Abständen miteinander verbindbaren Stellringen und
aus mit letzteren unter Längsspannung verbindbaren,
sich in den von den Stellringen aufgespannten Ebenen
erstreckenden und mit ihren Enden mittels Halterungen
an voneinander entfernten Teilen jeweils eines Stellrings befestigbaren Drähten zur Fixation der Knochenabschnitte oder -fragmente,
dadurch gekennzeichnet,
daß jeweils wenigstens eine der Halterungen zum Verbinden der Enden eines Drahtes (20,20', 58, 58')
mit einem Stellring (10,40) als in Drahtlängsrichtung
nachstellbares Spannelement (18,18', 38,38') ausgebildet
ist.

20.    Vorrichtung nach Anspruch 19, dadurch gekennzeichnet, daß das Spannelement (18,18') in einer
sich radial erstreckenden Führung (15,15') des Stellrings (10) bewegbar aufgenommen und mittels eines
Einstellgewindes längs der Führung nachstellbar ist.

21.    Vorrichtung nach Anspruch 20, dadurch gekennzeichnet, daß das Spannelement (18,18') verdrehfest
in der Führung (15,15') des Stellrings (10) aufgenommen
ist.

22.    Vorrichtung nach Anspruch 20 oder 21, dadurch
gekennzeichnet, daß jedes Spannelement (18,18') mit
einer Gewindespindel (23) versehen und auf letzterer
eine sich an einem Widerlager des Stellrings (10)
abstützende Spannmutter (25) aufgenommen ist.

23.    Vorrichtung nach einem der Ansprüche 19 bis 22,
dadurch gekennzeichnet, daß die Spannelemente (18,18') als Spannhülsen (22) mit einer sich radial zum Stellring erstreckenden Mittelbohrung (26) zum Aufnehmen
eines Drahtendes ausgebildet und mit Mitteln (28,28')
zum Festlegen der Drahtenden versehen sind.

24. Vorrichtung nach Anspruch 23, dadurch gekennzeichnet, daß als Mittel zum Festlegen der Drahtenden
wenigstens eine in einer rechtwinklig zur Mittelbohrung
(26) verlaufenden Gewindebohrung (27,27') aufgenommene
Klemmschraube (28,28') dient.

25. Vorrichtung nach Anspruch 24, dadurch gekennzeichnet,
daß in der Mittelbohrung (26) zwei in Bohrlängsrichtung
im Abstand voneinander angeordnete Gewindebohrungen
(27,27') mit Klemmschrauben (28,28') zum Festlegen
eines Drahtendes ausmünden.

26. Vorrichtung nach einem der Ansprüche 19 bis 25,
dadurch gekennzeichnet, daß der Stellring (10) in
zwei beabstandeten Parallelebenen mit in Umfangsrichtung in Abständen voneinander angeordneten Aufnahmen (15,15') für die Halterungen (18,18') zum
Verbinden der Drahtenden mit dem Stellring versehen
ist.

27. Vorrichtung nach Anspruch 26, dadurch gekennzeichnet, daß die Aufnahmen für die Halterungen (18,18')
sich radial durch den Stellring (10) erstreckende
Bohrungen (15,15') sind.

28. Vorrichtung nach Anspruch 26, dadurch gekennzeichnet, daß die Aufnahmen für die Halterungen (18,18')
den Stellring (10) radial durchdringende, jeweils zu einer Stellringstirnseite offene Ausnehmungen
sind.

29. Vorrichtung nach einem der Ansprüche 26 bis 28,
dadurch gekennzeichnet, daß der Stellring (10) als
im Querschnitt gesehen- T-Profil ausgebildet ist,
dessen Mittelsteg (11) sich in einer Ebene radial
nach außen erstreckt und bei dem die in beabstandeten
Parallelebenen angeordneten Aufnahmen (15,15') für
die Halterungen (18,18') sich in den Flanschteilen
(12, 12') beidseitig des Mittelstegs erstrecken.

30. Vorrichtung nach Anspruch 29, dadurch gekennzeichnet, daß der Stellring (10) einen sich in der
Ebene seines radial nach außen forterstreckenden
Mittelstegs (11) radial innenseitig erstreckenden
Ringflansch (13) aufweist.

31. Vorrichtung nach Anspruch 30, dadurch gekennzeichnet, daß die Spannelemente (18,18') auf der
zum Zentrum des Stellrings (10) hinweisenden Seite
einen hammerkopfartigen Ansatz (24) besitzen, der
mit wenigstens einer an dem innenseitigen Ringflansch
(13) des Stellrings anliegenden und eine Verdrehsicherung bewirkenden Flachseite (32) versehen ist.

32. Vorrichtung nach Anspruch 19, dadurch gekennzeichnet, daß die Spannelemente (38, 38') zum Verbinden
der Enden der Spanndrähte (58, 58') mit einem Stellring
als auf letzteren aufschiebbare und mittels Klemmung
an diesem Stellring festlegbare Halterungen (43, 43')
mit U-förmigen Teilen ausgebildet sind sowie sich

sowie sich an Widerlagern (55, 55') dieser Halterungen abstützende und nachspannbare Spannhülsen (44) aufweisen, in denen die Spanndrahtenden mittels Klemmung fest, jedoch lösbar verankert sind.

33. Vorrichtung nach Anspruch 32, dadurch gekennzeichnet, daß die Halterungen (43, 43') der Spannelemente (38, 38') in Umfangsrichtung der Stellringe auf diesen stufenlos verschiebbar und infolge Klemmung mit dem jeweiligen Stellring in jeder Einstellage arretierbar sind.

34. Vorrichtung nach Anspruch 32 oder 33, dadurch gekennzeichnet, daß die Spannelemente (38, 38') radial geführte und mittels sich an den Widerlagern (55,55') abstützender Spannmuttern (60) in Spannlage bringbare Spannhülsen (44) aufweisen, in denen infolge Klemmung die Enden von Spanndrähten (58, 58') festlegbar sind.

35. Vorrichtung nach Anspruch 34, dadurch gekennzeichnet, daß sich von einem Stirnende der Spannhülsen (44) jeweils eine Gewindespindel (53) mit einer auf dieser aufgeschraubten Spannmutter (60) fort- und durch eine Ausnehmung (54) in dem Widerlager der die Spannhülse aufnehmenden Halterung längsbeweglich hindurcherstreckt, wobei der mit seinem Ende in der Spannhülse festgelegte Spanndraht (58, 58') in einer Mittelbohrung (66) der Gewindespindel (53) aufgenommen und in der Spannhülse festgelegt ist und sich auf die auf die Gewindespindel (53) aufgeschraubte Spannmutter (60) radial außenseitig an dem Widerlager (55) abstützt.

36. Vorrichtung nach Anspruch 35, dadurch gekennzeichnet, daß sich von der Ausnehmung (54) ein das Widerlager (55, 55') seitlich durchbrechender Schlitz (56) forterstreckt, dessen Breite größer als der Durchmesser eines Spanndrahtes (58) aber kleiner als der Druchmesser einer Gewindespindel (43) ist.

37. Vorrichtung nach einem der Ansprüche 34 bis 36, dadurch gekennzeichnet, daß innerhalb einer sich von der von der Gewindespindel (53) entfernten Stirnseite aus in ein Mittelteil (52) der Spannhülse (44) hineinerstreckenden Bohrung (64), die in einer kegeligen Spannfläche (65) endet, eine spannfutterartige Klemmhülse (68) mit durch Längsschlitze (6) voneinander getrennten Klemmfingern aufgenommen ist, die eine ein eingeführtes Spanndrahtende aufnehmende Längsausnehmung aufweist und mittels einer in ein Innengewinde (70) der Bohrung (64) eingeschraubten Spannschraube (72) mit ihren Spannfingern infolge deren Zusammenwirkens mit der kegeligen Spannfläche (65) das Spanndrahtende durch Klemmung festlegt.

38. Vorrichtung nach einem der Ansprüche 32 bis 37, dadurch gekennzeichnet, daß beidseitig des radial auf einen Stellring (40) aufschiebbaren und an diesem befestigbaren U-förmigen Teils eines Spannelements (38') eine Radialführung mit einer in dieser aufgenommenen und jeweils an einem Widerlager abgestützten Spannhülse (44) angeordnet ist.

842312

0146872
-11-

39. Vorrichtung nach Anspruch 38, dadurch gekennzeichnet, daß die auf gegenüberliegenden Seiten eines
Stellringes (40) angeordneten Führungen für je eine
Spannhülse (44) parallel, divergierend oder konvergierend zueinander verlaufen.

0146872

Fig. 2  1|4

Fig. 1

Fig. 3

Fig. 4

Fig. 5

Fig 6

Fig. 7

Fig 9

Fig 10

Fig. 11

Fig 12

Fig. 13

Fig. 14

Fig. 15